# EUROPEAN PATENT APPLICATION

(11) **EP 1 559 317 A1**
(43) Date of publication of application: **03.08.2005**
(21) Application number: 03758961.1
(22) Date of filing: 28.10.2003
(51) Int. Cl.: A01K 67/027, A61K 45/00, A61P 13/08, G01N 33/15, G01N 33/50

(54) **NONBACTERIAL PROSTATITIS MODEL ANIMAL**

(30) Priority: 29.10.2002 JP 2002313960
(71) Applicant: TAIHO PHARMACEUTICAL COMPANY, LIMITED, Tokyo 101-0054 (JP)
(72) Inventor: NAKANO, Koushi, Taiho Pharmaceutical Co,Ltd., Tokushima-shi Tokushima 771-0194 (JP); KIRIMOTO, Tsukasa, Taiho Pharmaceutical Co.,Ltd., Tokushima-shi Tokushima 771-0194 (JP); HAYASHI, Yukio, Taiho Pharmaceutical Co., Ltd., Tokushima-shi Tokushima 771-0194 (JP); OKA, Toshinori Taiho Pharmaceutical Co., Ltd., Tokushima-shi Tokushima 771-0194 (JP)
(74) Representative: Jones, Helen Marjorie Meredith
(86) International application number: PCT/JP2003/013743
(87) International publication number: WO 2004/039148

(57) **Abstract**

The present invention provides an animal model for use in the development of a preventive or therapeutic agent for human chronic nonbacterial prostatitis, in particular a nonbacterial prostatitis nonhuman animal model mimicking the pathology of human chronic nonbacterial prostatitis. The nonbacterial prostatitis animal model is prepared by injecting hydrochloric acid beneath the prostatic capsule and exhibits prostate tissue damage and lower urinary tract disorders that are characteristically observed in human chronic nonbacterial prostatitis.

## Description

### TECHNICAL FIELD

The present invention relates to an animal model for chronic nonbacterial prostatitis and a method for producing it. More specifically, the invention relates to a nonhuman animal mimicking the pathology of human chronic nonbacterial prostatitis and a method for producing it. Moreover, the invention relates to a method using such an animal model for screening for a substance effective for preventing or treating human chronic nonbacterial prostatitis and to a pharmaceutical composition for preventing or treating human chronic nonbacterial prostatitis containing, as an active ingredient, a substance screened for by such a method. The invention is further directed to a method using such a model animal for evaluating the efficacy of a pharmaceutical composition for preventing or treating human chronic nonbacterial prostatitis.

### BACKGROUND OF THE INVENTION

Prostatitis is a urologic disease relatively common among adult males. In the United States, patients of prostatitis accounts for the largest proportion of patients of urological diseases among males aged 18 to 50 at 0.77% of the total outpatients, with patients of urological diseases accounting for about 5% of the total outpatients. Among males over 50 years old, prostatitis is reported as the third most common urologic disease (0.83% of total outpatients) after benign prostatic hyperplasia and prostate carcinomas (Collins M.M. How common is prostatitis? A national survey of physician visits. *J. Urol.* 159: 1224-1228, 1998). In the United States, about 2 million people each year are diagnosed with prostatitis.

In Japan, the number of new patients (people coming into primary care) of the prostatitis syndrome is estimated to be from about 600000 to about 1 million each year (Toshihiro Kitagawa, Hiroshi Hayami, Motoshi Kawahara, et al. Diagnosis and treatment of the prostatitis syndrome . Latest approach and trend. Patient number of the prostatitis syndrome. Does the prostatitis syndrome increase ?*.Prog. Med.* 1998, 18, 2149-2152). Prostatitis is roughly divided into 4 groups: (1) acute bacterial prostatitis (ABP), (2) chronic bacterial prostatitis (CBP), (3) chronic nonbacterial prostatitis (CNP), and (4) prostatodynia. The type-specific incidence of prostatitis is reported to be rare for bacterial prostatitis (1) and (2), and nonbacterial prostatitis (3) and (4) accounts for most incidences of prostatitis (Soichi Arakawa, Causes and therapy for prostatitis. *Japanese Medical Journal* 4017: 25-30, 2001). In terms of clinical pathology, prostatitis is roughly categorized into acute: (1), and chronic: (2), (3) and (4). Symptoms of acute bacterial prostatitis (1) categorized as an acute disease include, in addition to fever, so-called bladder symptoms derived from the spreading of inflammation from the bladder trigone to the posterior urethra, such as urodynia, pollakiuria and a sense of residual urine. Subjective symptoms of chronic prostatitis (2), (3) and (4) categorized as a chronic disease include bladder symptoms such as pollakiuria and urodynia, and unidentified complaints about the perineum and the hypogastrium. Pollakiuria is one of the typical symptoms that are perceived relatively commonly (Arakawa Soichi: Prostatitis and pollakiuria. *Kongetsu no chiry* 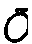 6: 863-865, 1998). As described above, chronic prostatitis is categorized into 3 groups: (2), (3) and (4). However, the clinical pathologies thereof are very similar, and therefore it is difficult to identify the type of prostatitis based on clinical pathology (Masayoshi Yokoyama, All of prostatic gland medical care. Prostatitis and prostatodynia. *The Journal of Clinical Science* 33: 1561-1567, 1997).

An example of a method for prostatitis diagnosis is to identify the type of prostatitis based on the result of the four-glass test. Although the four-glass test should not be performed with respect to (1) acute bacterial prostatitis (ABP) due to the risk of septicemia, the four-glass test with respect to chronic prostatitis (2), (3) and (4) can identify bacterial and nonbacterial prostatitis through the examination of the presence/absence of bacteria in EPS (expressed prostatic secretion, fluid obtained during prostate massage) and VB3 (voided bladder 3, the first urine after prostate massage). Moreover, nonbacterial prostatitis and prostatalgia can be determined by the presence/absence of leucocytes in EPS (Masayoshi Yokoyama, (All of prostatic gland medical care. Prostatitis and prostatodynia. *The Journal of Clinical Science* 33: 1561-1567, 1997). Recently, the National Institutes of Health chronic prostatitis symptom index (NIH-CPSI) has been published by the NIH as a method for diagnosing chronic prostatitis. The NIH-CPSI grades the symptoms of chronic prostatitis characterized by various clinical symptoms as symptomatic scores. The NIH-CPSI regards pain to be the trait of the prostatitis syndrome. Irritative symptoms such as pollakiuria, urinary incontinence, nycturia, and urgency of urination are emphasized more in the QOL (quality of life) score than obstructive symptoms such as urination difficulty, uroschesis, prolonged urination, and intermittent urination (Soichi Arakawa Causes and therapy for prostatitis. *Japanese Medical Journal,* 4017: 25-30, 2001).

The NIH-CPSI is used in the United States and Japan because it is considered important for determining the severity of chronic prostatitis and the efficacy of medication. Based on researches conducted using the NIH-CPSI, it is reported that chronic prostatitis, when compared with prostatic hyperplasia, which is another prostatic disorder, exhibits similar scores in the urinary irritation symptom domain (such as pollakiuria, urinary incontinence, nycturia, and urgency of urination) but higher scores in the pain/discomfort and QOL (quality of life) domains (Koichi Monden, Masaya Tsugawa, Yuko Ninomiya, et al., A Japanese Version of The National Institutes of Health Chronic Prostatitis Symptom Index (NIH-CPSI, Okayama Version) and the Clinical Evaluation of Cernitin Pollen Extract for Chronic Non-bacterial Prostatitis. *The Japanese Journal of Urology* 93: 539-547, 2002; Mark, SL., Mary, M-C., Floyd, JF. et al., The National Institutes of Health chronic prostatitis symptom index: Development and validation of new outcome measure. *J. Urol*. 162: 369-375, 1999). Therefore, it can be understood that the subjective symptoms of chronic prostatitis include considerable discomfort. The NIH-CPSI score reacknowledges that the end point of the treatment of the chronic prostatic syndrome should be the remission of symptoms (amelioration of dysuria, pain, QOL).

The etiology of chronic nonbacterial prostatitis takes into consideration various factors such as retention of the prostatic fluid; involvement of autoimmune systems, allergy, and microorganisms such as *Mycoplasma, Chlamydia trachomatis,* etc.; imbalance of sex hormones; psychological aspects; etc. (Pewitt, E.B., Schaeffer, A.J. Urinary tract infection in urology, including acute and chronic prostatitis. *Infect. Dis. Clin. North Am*. 11: 623-646, 1997; Donovan, D.A., Nicholas, P.K. Diagnosis and treatment in primary care. *Nurse Practitioner* 22: 144-156, 1997). Therefore, various treatments have been attempted such as the administration of antibacterial agents; prostate massage; the administration of α1 blockers (prazocin, bunazocin, terazocin, etc.), sedative-hypnotic drugs , and antianxiety agents (diazepam and the like) for the amelioration of dysuria; the administration of plant extracts, pollen extracts(cernilton), Chinese medicine formulations, nonsteroidal antiphlogistic sedative agents, etc., for removal of edemas at the prostatic urethra; warm sitz bath; hyperthermia; etc (Shoichi Onodera, Diagnosis and treatment of the prostatitis syndrome. Latest approach and trend. The treatment according to the pathophysiology of the prostatitis syndrome. 4. The non-bacterial prostatitis. *Prog. med.* 1998, 18, 2191-2194). However, chronic nonbacterial prostatitis is a persistent intractable disease exhibiting resistance to such various treatments. The reason therefor would be considered as follows, since chronic nonbacterial prostatitis is caused by many different factors, even when inflammation of the prostate, which is the primary factor in causing its symptoms, is ameliorated by the administration of antibacterial agents, anti-inflammatory agents, etc., the influence of prostatitis on the bladder, urethra, etc., situated adjacent to the prostatic tissue (hereinafter the bladder and the urethra are sometimes collectively referred to as the "lower urinary tract") and on the nervous system involved in urination may remain, thereby possibly causing pain and irritative voiding symptoms (Yasushi Tsukamoto, Naoya Masumori, Clinical prostate gland. Symptoms of voidance and the prostate gland. *The Journal of Japan Medical Association* 119: 609-612, 1998).

There are many diseases that cause lower urinary tract disorders as their clinical pathologies. Among lower urinary tract disorders, examples of diseases that cause irritative voiding symptoms (urine storage disorders) such as pollakiuria, urinary incontinence, etc., are reported to be cystitis, urethritis, prostatitis, nervous pollakiuria, bladder neurosis, contracted bladder (interstitial cystitis and the like), etc. Examples of diseases that cause obstructive voiding symptoms (dysuria) such as urination difficulty, ischuria, etc., are reported to be bladder detrusor muscle contraction disorder, bladder neck sclerosis, bladder tumor, prostate carcinoma, prostatitis, urethrostenosis, urethrophyma, etc (Ryuichiro Miyatake, Takashi Kurita, Prostate Hyperplasia: Differential Diagnosis of Dysuria of Other Causes. *Dysurea Practice* 2002, 10, 78-81). As described above, prostatitis may pose both irritative voiding symptoms (urine storage disorders) and obstructive voiding symptoms (dysuria) of the lower urinary tract disorders. Therefore, a novel method and a novel agent for treating prostatitis including ameliorating such lower urinary tract disorders are desired.

Production of various prostatitis animal models have been attempted and examined in basic researches as a means for identifying pathologies of chronic prostatitis and developing therapeutic agents therefor. An example of animal model production for bacterial prostatitis is direct inoculation with an infecting organism. Principal examples of animal model production for nonbacterial prostatitis are production of such animal model by using autoimmune reaction and production of such animal model by inducing hormonal imbalance by excessive administration of androgen and estrogen. Other examples of methods for animal model production include those employing partial urinary tract obstruction by surgical means; spontaneous development by aging; stressful stimuli; direct inoculation of lipopolysaccharide (LPS) into the prostate; drug administration to female rats during pregnancy and lactation to spontaneously induce prostatitis in infant male rats; continuous feeding of modified food; inoculation of an infecting microorganism (chlamydia); etc. Researches conducted on such animal models has focused mainly on pathohistological examination of the inflamed prostate, fluctuation of inflammatory cytokine levels accompanying therewith, parameters for inflammatory cellular infiltration (mast cell, lymphocyte), changes in inflammation-responsible genes, etc. There has been no research report focusing on subjective symptoms (urination difficulty, pain, QOL) of concern in a clinical environment.

When investigating pathologies accompanied by prostatitis, if an animal model that reflects the clinical pathologies of prostatitis, having both prostatic tissue damage and lower urinary tract disorders, can be produced, effects on the amelioration of prostatic tissue damage and lower urinary tract disorders can be simultaneously evaluated using a single animal model. Such an animal model is therefore expected to be highly useful in the screening of active ingredients and efficacy (as an animal model for screening) in the development of preventive/therapeutic agents for prostatitis.

As lower urinary tract disorders accompanied by chronic prostatitis, pollakiuria (urine storage disorder) is observed in nonbacterial prostatitis due to edema and swelling by inflammation, and urination difficulty (dysuria) is observed in prostatodynia due to the hypertonic pelvic floor muscle, dysfunction of the lower urinary tract, etc. Such lower urinary tract disorders are known to be brought about by the presence of the prostate, which is anatomically located such that the prostate encloses the urethra at the outlet of the bladder. Therefore, prostatic diseases such as prostatitis are likely to accompany lower urinary tract disorders to varying extents. Moreover, it has been pointed out that prostatic diseases cause lower urinary tract disorders and sometimes lead to alteration in form and function of the bladder (*Dysurea Practice* 8: 41-48, 2000).

However, there are currently no nonhuman animal pathological models that exhibit lower urinary tract disorders (urine storage disorders such as pollakiuria, and dysuria such as urination difficulty) resulting from prostatitis as observed in human chronic prostatitis. Therefore, the development of such animal models is required.

Examples of known nonbacterial prostatitis animal models are (1) an animal model prepared with use of lipopolysaccharide (LPS) (Fulmer, B.R., Turner, T.T. Effect of inflammation on prostatic protein synthesis and luminal secretion in vitro. *J. Urol*. 162: 248, 1999); (2) an animal model prepared with use of dihydrotestosterone + estradiol (Harris, M.T., Feldberg, R.S., Lau, K.M. et al. Expression of proinflammatory gene during estrogen-induced inflammation of the rat prostate. *The Prostate* 44: 19-25, 2000); (3) an animal model prepared by injecting estradiol to castrated rats (Robinette, C.L. Sex-hormone-induced inflammation and fibromuscular proliferation in the rat laternal prostate. *The Prostate* 12: 271-286, 1988); (4) an animal model prepared by instilling ethanol into the ventral prostates of male rats to reduce mucosal integrity and adding dinitrobenzenesulfonic acid (DNBS) to induce inflammation (Micheal, DL., J.Curtis, N., Merle, EO. et al.: Rat model of experimentally induced abacterial prostatitis. *The Prostate* 45: 201-206, 2000); and (5) an animal model prepared by injecting hydrochloric acid into the vas deferens of rats (Toshihiro Goto, *Clinical and Experimental Studies on Prostatitis Nishinihon J. Urol. 50: 446-455, 1988). However, it has been pointed* out that animal models (1) to (4) have disadvantages of varying extent as follows. In particular, prostatic tissue damage of animal model (1) are limited to the site of LPS injection and the periphery thereof. It takes a relatively long period of time (at least 3 to 4 weeks) to prepare animal models (2) and (3) by creating a hormonal imbalance by administration of hormones; and it is difficult to confirm that hormones are securely administered during animal model preparation and therefore it is possible that animal model preparation fails due to hormone administration capsule problems. During preparation of animal model (3), two operations are needed for castration and hormone capsule implantation, thereby complicating the procedure. Animal model (4) undergoes severe prostatitis such that more than half of the model animals die of urethral obstruction by the 7^{th} day of preparation. Experiments conducted by the inventors revealed that, from the pathohistological point of view, animal model (5) develop prostatitis only to a very slight extent (see examples below).

### DISCLOSURE OF THE INVENTION

An object of the present invention is to provide a (nonhuman) animal model effectively usable in the development of a preventive or therapeutic agent for human chronic nonbacterial prostatitis. In particular, an object of the invention is to provide a nonbacterial prostatitis (nonhuman) animal model mimicking the pathology of human chronic nonbacterial prostatitis in terms of pathohistological change in the prostate and presence/absence of lower urinary tract disorders, and preferably in terms of bladder weight increase.

Further, another object of the present invention is to provide a method for producing such an animal model.

Still another object of the present invention is to provide a method for screening for a substance effective for preventing or treating human chronic nonbacterial prostatitis using such an animal model, and to provide a pharmaceutical composition for preventing or treating human chronic nonbacterial prostatitis containing as an active ingredient a substance obtained according to such a method.

Yet another object of the invention is provide a method for evaluating the efficacy of a test drug on the prevention of human chronic nonbacterial prostatitis, and a method for evaluating the efficacy of a test drug on the treatment of human chronic nonbacterial prostatitis using such an animal model.

The inventors conducted extensive research to solve the problems described above and found that a nonhuman animal having a symptom similar to the pathology of human chronic nonbacterial prostatitis in terms of pathohistological change in the prostate and presence/absence of lower urinary tract disorders, and preferably in terms of bladder weight increase, can be prepared by injecting hydrochloric acid to a nonhuman animal beneath the prostatic capsule. The inventors were convinced that using the nonhuman animal as an animal pathological model of human chronic nonbacterial prostatitis a drug (candidate substance) effective for prevention or treatment of human chronic nonbacterial prostatitis can be obtained and the pharmaceutical efficacy of such a drug for the prevention or treatment of human chronic nonbacterial prostatitis can be evaluated. The present invention has been accomplished based on such findings.

In particular, the present invention provides animal models for nonbacterial prostatitis as described in Items (1) to (6) below:
(1) A nonbacterial prostatitis animal model exhibiting a tissue damage in the prostate tissue or the prostate and the tissue in the vicinity thereof that is characteristically observed in human chronic nonbacterial prostatitis and a lower urinary tract disorder that is characteristically observed in human chronic nonbacterial prostatitis,
the animal model being a nonhuman animal and being prepared by injecting hydrochloric acid beneath the prostatic capsule.
(2) The nonbacterial prostatitis animal model according to Item (1) having an increased ratio of bladder weight to body weight (bladder weight/body weight) compared with a normal nonhuman animal.
(3) The nonbacterial prostatitis animal model according to Item (1) or (2), wherein the lower urinary tract disorder is a urine storage disorder posing at least one symptom selected from the group consisting of pollakiuria, urinary incontinence, and reduced effective bladder capacity (reduced volume of urine per urination).
(4) The nonbacterial prostatitis animal model according to any one of Items (1) to (3), wherein the animal model is a nonhuman animal, and prepared by injecting hydrochloric acid beneath the capsule of a dorsolateral lobe.
(5) The nonbacterial prostatitis animal model according to any one of Items (1) to (4), wherein the animal model is a nonhuman animal, and reared for about 4 days to about 1 week after injection of hydrochloric acid beneath the prostatic capsule.
(6) The nonbacterial prostatitis animal model according to any one of Items (1) to (5), wherein the nonhuman animal is a rat.

Furthermore, the present invention provides methods for preparing the aforementioned nonbacterial prostatitis animal models as described in Items (7) to (10) below:
(7) A method for preparing the nonbacterial prostatitis animal model recited in any one of Items (1) to (3) comprising the steps of:
   injecting hydrochloric acid beneath the prostatic capsule of a nonhuman animal, and
   rearing the nonhuman animal to develop prostatitis.
(8) The method for preparing the nonbacterial prostatitis animal model according to Item (7), wherein hydrochloric acid is injected beneath the prostatic capsule of a dorsolateral lobe.
(9) The method for preparing the nonbacterial prostatitis animal model according to Item (7) or (8) comprising the steps of:
   injecting hydrochloric acid beneath the prostatic capsule, and
   rearing the nonhuman animal for about 4 days to about 1 week.
(10) The method for preparing the nonbacterial prostatitis animal model according to any one of Item (7) to (9), wherein the nonhuman animal is a rat.

Moreover, the present invention provides methods for screening for a substance for treating human chronic nonbacterial prostatitis as described below:
(11) A method for screening for a substance for treating human chronic nonbacterial prostatitis comprising the steps of:
   administering a test substance to the nonbacterial prostatitis animal model according to any one of Items (1) to (6), and
   examining the effect of the test substance on the amelioration of at least one disorder selected from a prostate tissue damage and a lower urinary tract disorder in the nonbacterial prostatitis animal model.
(12) The method for screening for a substance for treating human chronic nonbacterial prostatitis according to Item (11), wherein the lower urinary tract disorder is a urine storage disorder posing at least one symptom selected from the group consisting of pollakiuria, urinary incontinence, and reduced effective bladder capacity (reduced volume of urine per urination).
(13) The method for screening for a substance for treating human chronic nonbacterial prostatitis according to Item (11) or (12), wherein the nonbacterial prostatitis animal model is a rat.

Furthermore, the present invention provides methods for screening for a substance for preventing human chronic nonbacterial prostatitis as described below:
(14) A method for screening for a substance for preventing human chronic nonbacterial prostatitis comprising the steps of:
   administering a test substance to a preparatory nonbacterial prostatitis animal model prepared by injecting hydrochloric acid beneath the prostatic capsule, which has not yet developed prostatitis, and
   examining the effect of the test substance for inhibiting the development of at least one disorder selected from a prostate tissue damage and a lower urinary tract disorder of the preparatory nonbacterial prostatitis animal model.

The preparatory nonbacterial prostatitis animal model used in Item (14) is a nonhuman animal that has not yet developed prostatitis, and can be prepared in the same manner as the method for producing a nonbacterial prostatitis animal model according to any one of Items (7) to (10) except that the nonhuman animal has been reared for less than 4 days.

The methods of Items (11) to (13) and (14) can be used as methods for evaluating the therapeutic effects and preventive effects of test substances (test drugs) on human nonbacterial prostatitis. Such methods can be defined as in Items (15) to (16) and (17) to (18):
(15) A method for evaluating the efficacy of a test drug for amelioration of human nonbacterial prostatitis, the method comprising the steps of:
   administering the test drug to be examined for the efficacy to the nonbacterial prostatitis animal model according to any one of Items (1) to (6), and
   examining the effect of the drug for ameliorating at least one disorder selected from a prostate tissue damage and a lower urinary tract disorder in the nonbacterial prostatitis animal model.
(16) The method for evaluating efficacy of a drug on the amelioration of human nonbacterial prostatitis according to Item (15), wherein the lower urinary tract disorder is a urine storage disorder posing at least one symptom selected from the group consisting of pollakiuria, urinary incontinence, and reduced effective bladder capacity (reduced volume of urine per urination).
(17) A method for evaluating the efficacy of a drug for preventing human nonbacterial prostatitis, the method comprising the steps of:
   administering a the drug to be tested for the efficacy to a preparatory nonbacterial prostatitis animal model produced by injecting hydrochloric acid beneath the prostatic capsule and that having not developed prostatitis, and
   examining the effect of the drug for inhibiting the development of at least one disorder selected from a prostate tissue damage and a lower urinary tract disorder of the preparatory nonbacterial prostatitis animal model.
(18) The method for evaluating the efficacy of a drug for preventing human nonbacterial prostatitis according to Item (17), wherein the lower urinary tract disorder is a urine storage disorder posing at least one symptom selected from the group consisting of pollakiuria, urinary incontinence, and reduced effective bladder capacity (reduced volume of urine per urination).

The preparatory nonbacterial prostatitis animal model used in Item (17) is a nonhuman animal that has not yet developed prostatitis, and can be obtained in the same manner as the method for producing a nonbacterial prostatitis animal model recited in any one of Items (1) to (6) above except that the nonhuman animal is reared for less than 4 days.

Further, the present invention is directed to pharmaceutical compositions containing substances obtained by the screening methods according to Items (11) or (14):
(19) A pharmaceutical composition for treating or preventing human nonbacterial prostatitis comprising a substance evaluated as having an effect of ameliorating or preventing nonbacterial prostatitis by the screening method recited in any one of Items (11) or (14).

The present invention includes the following modes:
(20) Use of the nonbacterial prostatitis animal model according to any one of Items (1) to (6) for searching for an active ingredient for a pharmaceutical composition for treating human nonbacterial prostatitis or for evaluating the efficacy of a drug for treating human nonbacterial prostatitis.
(21) Use of a preparatory nonbacterial prostatitis animal model prepared by injecting hydrochloric acid beneath the prostatic capsule, which has not yet developed prostatitis, for searching for an active ingredient for a pharmaceutical composition for preventing human nonbacterial prostatitis or for evaluating the efficacy of a drug for preventing human nonbacterial prostatitis.
(22) The use according to Item (21), wherein the preparatory nonbacterial prostatitis animal model is a nonhuman animal that has not yet developed prostatitis, the animal model being prepared by injecting hydrochloric acid beneath the prostatic capsule of a nonhuman animal and rearing the nonhuman animal for less than 4 days.

### BRIEF DESCRIPTION OF THE DRAWINGS

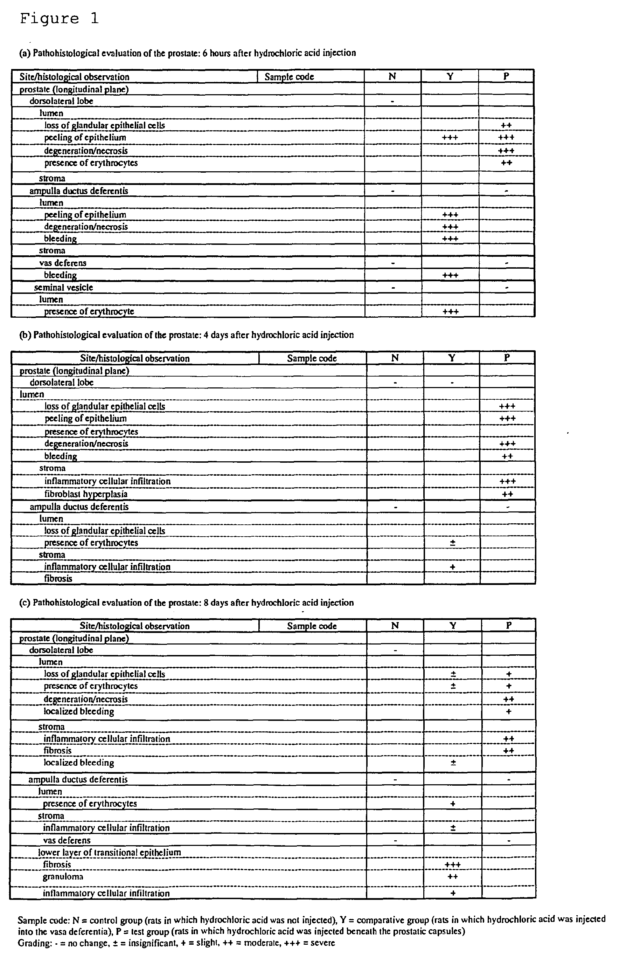

Figure 1 tabulates observations, in 2) of Example 1, of tissues at the sites of hydrochloric acid injection (pathohistological evaluation of the prostates) of rats in which hydrochloric acid was injected beneath the prostatic capsule (test group: P), rats in which hydrochloric acid was injected into the vas deferens (comparative group: Y), and rats in which hydrochloric acid was not injected (control group: N), when viewed along the longitudinal plane (the plane created by placing the tissue such that the urethra came at the center, and then longitudinally cutting the tissue (the median line of a rat extending from the head to the tail is regarded as the longitudinal direction)). (a) shows observations of prostate tissues of rats 6 hours after hydrochloric acid injection. (b) shows observations of prostate tissues of rats 4 days after hydrochloric acid injection. (c) shows observations of prostate tissues of rats 8 days after hydrochloric acid injection.

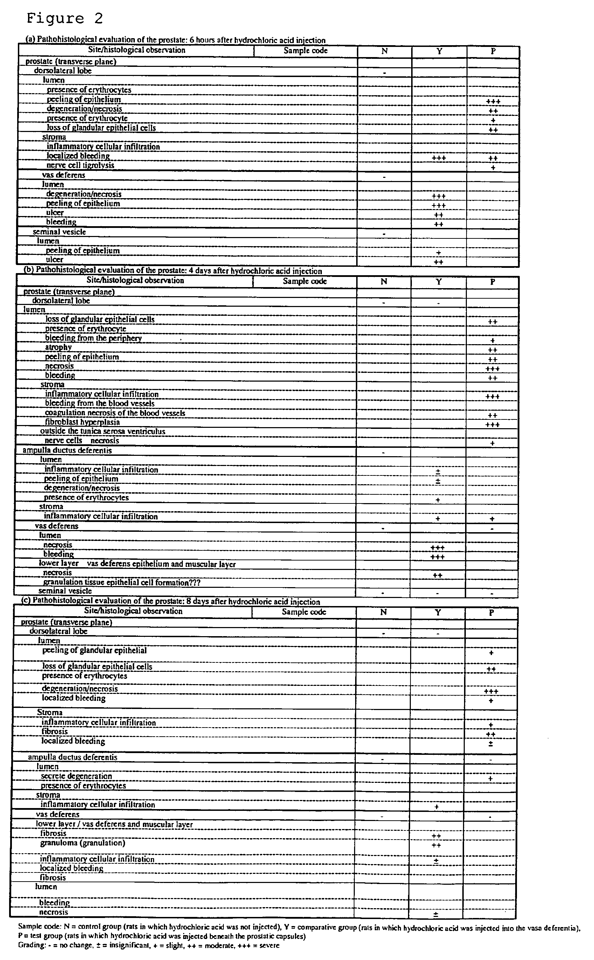

Figure 2 tabulates observations, in 2) of Example 1, of tissues at the sites of hydrochloric acid injection (pathohistological evaluation of the prostates) of rats in which hydrochloric acid was injected beneath the prostatic capsule (test group: P), rats in which hydrochloric acid was injected into the vas deferens (comparative group: Y), and rats in which hydrochloric acid was not injected (control group: N), when viewed along the transverse plane (the plane created by the placing the tissue such that the urethra came at the center, and then transversely cutting the tissue (the median line of a rat extending from the head to the tail is regarded as the longitudinal direction)). (a) shows observations of prostate tissues of rats 6 hours after hydrochloric acid injection. (b) shows observations of prostate tissues of rats 4 days after hydrochloric acid injection. (c) shows observations of prostate tissues of rats 8 days after hydrochloric acid injection.

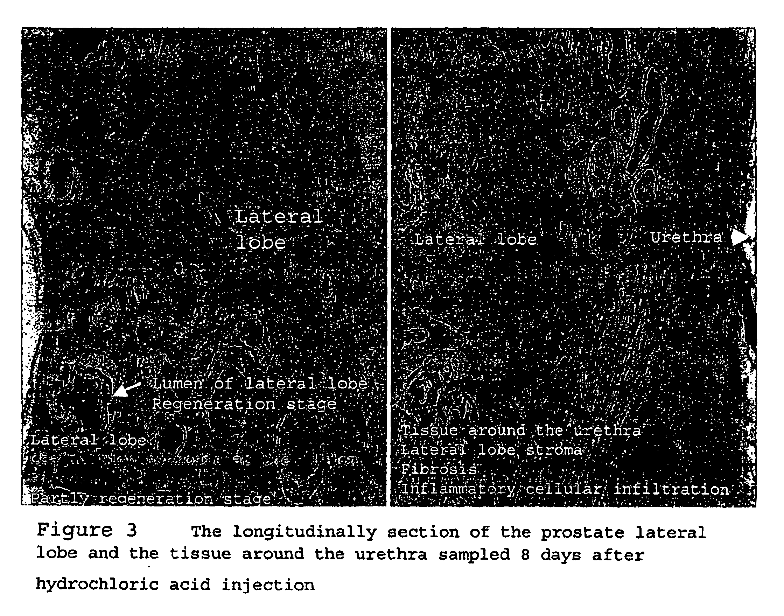

Figure 3 shows longitudinally sectional images of the prostate lateral lobe and the tissue around the urethra of the prostate tissue sampled 8 days after hydrochloric acid injection from the rats to which hydrochloric acid had been injected beneath the prostatic capsule (see 2-1) of Example 1).

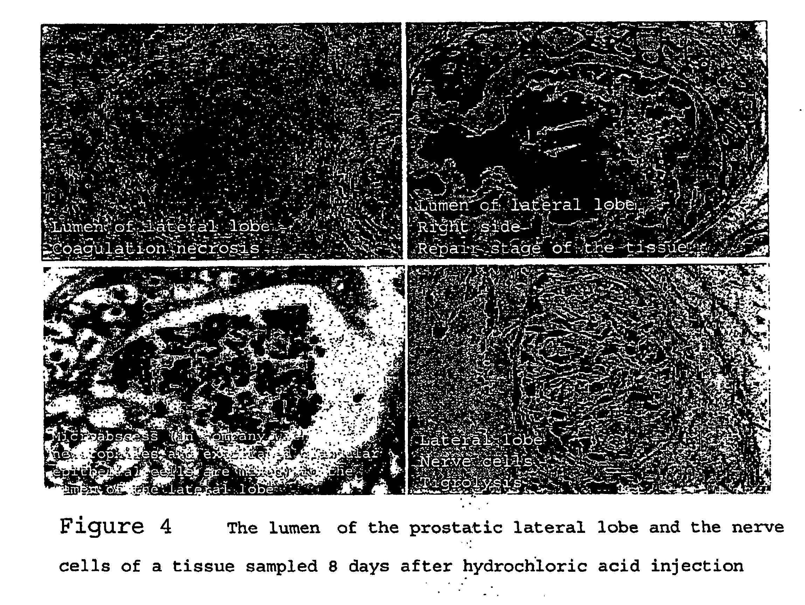

Figure 4 shows images of the lumen of the prostate lateral lobe and the nerve cells of prostate tissue sampled 8 days after hydrochloric acid injection from the rats to which hydrochloric acid had been injected beneath the prostatic capsule (see 2-1) of Example 1).

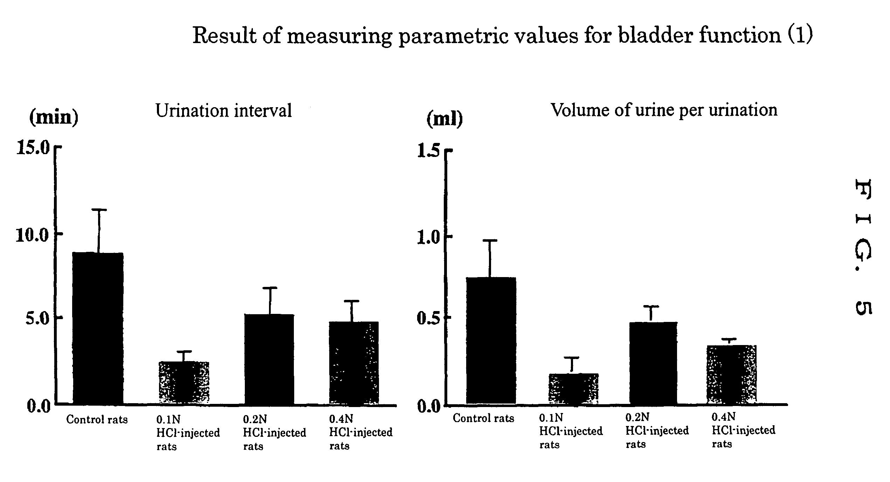

Figure 5 shows the experimental results of 3) of Example 1, i.e., charts showing the urination intervals and volumes of urine per urination of the rats to which hydrochloric acid was injected beneath the prostatic capsule (0.1 N hydrochloric acid-injected rats, 0.2 N hydrochloric acid-injected rats, and 0.4 N hydrochloric acid-injected rats, at the 8^{th} day of rearing after hydrochloric acid injection) and of the rats of the control group.

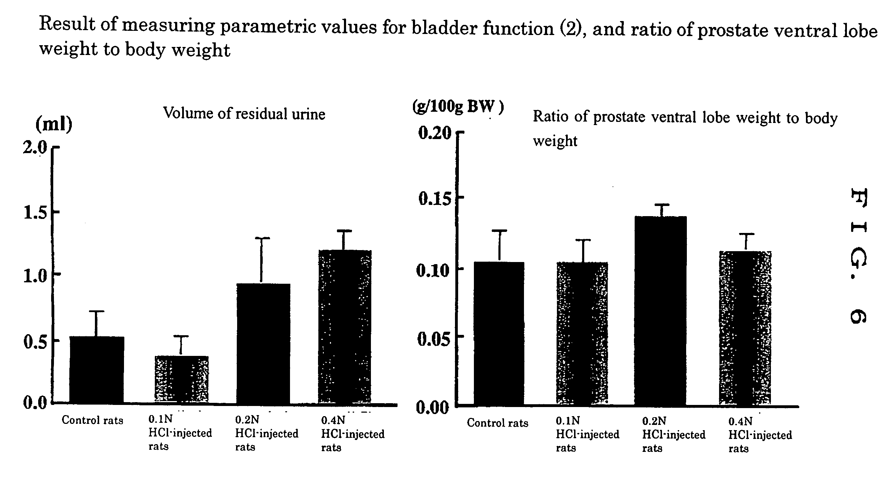

Figure 6 shows the experimental results of 3) of Example 1, i.e., charts showing the volumes of residual urine and the ratios of prostate ventral lobe weight to body weight of the rats to which hydrochloric acid was injected beneath the prostatic capsule (0.1 N hydrochloric acid-injected rats, 0.2 N hydrochloric acid-injected rats, and 0.4 N hydrochloric acid-injected rats, at the 8^{th} day of rearing after hydrochloric acid injection) and of the rats of the control group.

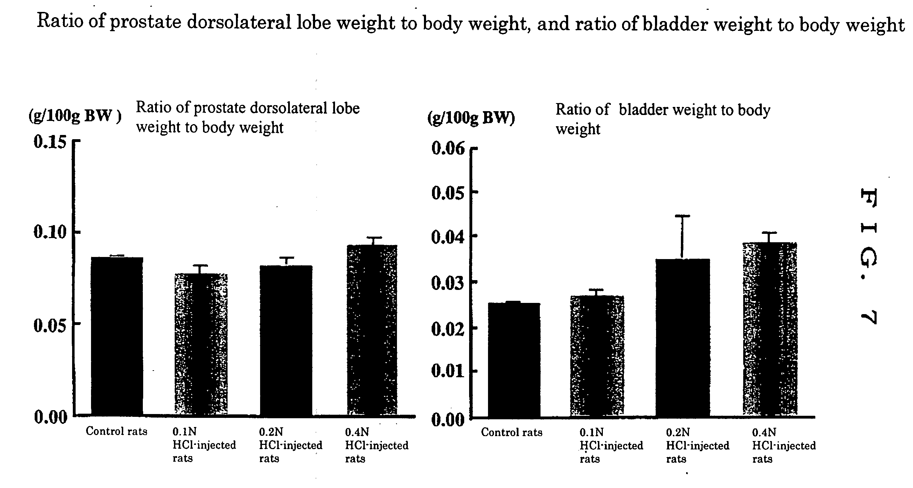

Figure 7 shows the experimental results of 3) of Example 1, i.e., charts showing the ratios of the prostate dorsolateral lobe weight to body weight and the ratios of bladder weight to body weight of the rats to which hydrochloric acid was injected beneath the prostatic capsule (0.1 N hydrochloric acid-injected rats, 0.2 N hydrochloric acid-injected rats, and 0.4 N hydrochloric acid-injected rats, at the 8^{th} day of rearing after hydrochloric acid injection) and of the rats of the control group.

### BEST MODE FOR CARRYING OUT THE INVENTION

### I. Nonbacterial prostatitis animal model and method for producing it

The nonbacterial prostatitis animal model to which the present invention is directed is an animal (excluding humans) pathological model (i.e., of a nonhuman animal) reflecting human chronic nonbacterial prostatitis. In particular, the nonbacterial prostatitis animal model of the present invention is a nonhuman animal in which at least the prostate tissue, and preferably both the prostate and the tissue in the vicinity thereof, exhibits pathohistological characteristics observed in the prostate and the tissue in the vicinity thereof of a human suffering from chronic nonbacterial prostatitis and lower urinary tract disorders as characteristically observed in human chronic nonbacterial prostatitis.

It is reported that with respect to pathohistological characteristics of human chronic nonbacterial prostatitis, histological image shows, chronic inflammatory response and stromal strand hyperplasia and the infiltration of lymphocytes, plasma cells and macrophages at the periphery of a lobule is observed, often accompanying ductal basal cell hyperplasia(*Surgical Pathology,* Bunkodo, 1999, 793-794). The nonbacterial prostatitis animal model to which the present invention is directed, in particular, exhibits characteristics such as inflammatory cellular infiltration and connective tissue increase (hyperplasia) in at least the interstitium of the prostate.

Although details are presented in the Example below, in the preferable animal model of the present invention, severe fibroblast hyperplasia and fibrosis are observed in the stroma of the prostate at least 4 days after animal model production treatment (hydrochloric acid injection), and, inflammatory cellular infiltration (infiltration of macrophages, eosinophilic leucocytes, etc.). The pathohistology of the animal model is in agreement with that of human chronic nonbacterial prostatitis. In the animal model, the aforementioned damage can be observed even in portions of prostate tissue in the vicinity of the urethra, and such tissue damage remain for 8 days or longer after animal model production treatment (hydrochloric acid injection). The animal model, however, suffers from damage only in the prostate and the tissue in the vicinity thereof, and does not exhibit urethra or bladder tissue damage. Therefore, the preferable nonbacterial prostatitis animal model of the present invention is a nonhuman animal that exhibits damage in the prostate and/or the tissue in the vicinity thereof similar to those characteristically observed in human chronic nonbacterial prostatitis, but does not have tissue damage in other lower urinary tract tissues such as urethral and bladder tissues.

With respect to the pathology of human chronic nonbacterial prostatitis, pain in the lower abdomen and perineum, unpleasant sensation during ejaculation, and lower urinary tract disorders (symptoms concerning urination: urine storage disorders such as pollakiuria, and dysuria such as urination difficulty) are reported. For diagnosis of chronic nonbacterial prostatitis, interview sheets are used in which scores are given according to the urinary symptoms and the pain in the regions of the urinary organs, genital organs and pelvis. The symptoms most typical among chronic nonbacterial prostatitis patients are urinary symptoms, particularly frequent urination (urine storage disorder: the symptom of shortened urination intervals). This is caused by the creation of inflammation in the prostate, thereby overactivating the bladder (uninhibited bladder contraction, unstable bladder). Normally, perception of the bladder is transmitted to the spinal cord via the Aδ fiber. However, a study shows that the C fiber is likely to serve as an injury signal transmitting nerve during inflammation, and perception of the bladder is transmitted by the C fiber as well as the Aδ fiber, thereby inducing hyperesthesia and resulting in uninhibited bladder contraction (*Dysurea Practice* 2000, 8, 97-102). In addition, such unstable bladder is accompanied by changes in the nervous system (*Dysurea Practice* 2000, 8, 97-102).

The nonbacterial prostatitis animal model to which the present invention relates is a nonhuman animal having at least a lower urinary tract disorder among the aforementioned pathological conditions (symptoms) characteristically observed in human chronic nonbacterial prostatitis. Among lower urinary tract disorders, preferable are urine storage disorders having symptoms such as pollakiuria, urinary incontinence, and reduced effective bladder capacity (reduced volume of urine per urination).

Although details are presented in the Example below, the preferable animal model of the present invention gives a shorter urination interval, a smaller volume of urine per urination, and a larger volume of residual urine than corresponding normal nonhuman animals. The pathology (symptoms) of the animal model is identical to the pathology (symptoms) of lower urinary tract disorders of human patients suffering chronic nonbacterial prostatitis. Therefore, the preferable nonbacterial prostatitis animal model of the present invention can be a nonhuman animal having lower urinary tract disorders characteristically observed in human chronic nonbacterial prostatitis, and in particular urine storage disorders having at least pollakiuria, reduced effective bladder capacity (reduced volume of urine per urination) or increased volume of residual urine.

With respect to nonhuman animals, examples of methods for evaluating the aforementioned urine storage disorders, in particular, methods that evaluate pollakiuria, effective bladder capacity (volume of urine per urination), and residual urine are those that measure urination interval, volume of urine per urination, and volume of residual urine. Such methods have already been established in this field of art as "The measuring method of intravesical pressure and micturition volume simultaneously in awake rats", "Cystometry" etc.(*Am. J. Physiol.* 1986, 251, R1177; *Experimental methods for neurourological researches.* Nihon-Igakukan Co., Ltd., 2000, 158-165). Evaluation of urine storage disorders can be carried out according to such methods.

Although details are presented in the Example, such methods can be briefly described as follows:
1. A subject nonhuman animal is weighed and anesthesized by intraperitoneally administering urethane, which is an anesthetic, in an amount of 1.0 g/kg. The abdomen is incised to expose the bladder.
2. A polyethylene catheter is inserted into the bladder of the nonhuman animal to the depth of about 6 mm and fixed.
3. The tip of the catheter opposite to that inserted into and fixed in the bladder is divided by a three-way cock, and one opening thereof is connected to a pressure amplifier via a transducer to measure the inner pressure of the bladder.
4. The other opening is connected via a physiological saline warmer to a continuous infuser filled with physiological saline for the introduction of physiological saline.
5. The subject nonhuman animal is secured in the prone position. Physiological saline (37°C) is introduced into the bladder at a rate of 5.0 ml/hr, and the inner pressure of the bladder is measured and recorded over time.
6. Changes in the volume of urine (voided volume) excreted from the bladder through the urinary duct are measured and recorded using a digital scale provided immediately below the cage housing the nonhuman animal and a Petri dish continually trapping the excreted urine.
7. The fluid remaining in the bladder at the end of urination is suctioned to give the volume of residual urine.
8. Urination interval and volume of urine per urination are computed by analyzing the result of monitoring the waveform of the inner pressure of the bladder and the volume of urine for 30 minutes (from 30 minutes after the beginning of measurement to 60 minutes after the beginning of measurement).

Recently, measurement of the weight of the bladder is reported to be advantageous in a clinical environment for predicting ischuria and estimating the extent of obstruction of the lower urinary tract and histological change of the bladder (*Dysurea Practice* 2000, 8, 41-48). Therefore, in the future the weight of the bladder will presumably be a useful factor for predicting the pathology of lower urinary tract disorders commonly observed in prostatic diseases including nonbacterial prostatitis. Therefore, more preferable, the nonbacterial prostatitis animal model of the present invention exhibits the aforementioned histological (prostate tissue damage) and pathological characteristics (lower urinary tract disorders) as observed in human chronic nonbacterial prostatitis and, as an additional feature, has an increased bladder weight compared with corresponding normal (healthy) nonhuman animals. An increase in the weight of the bladder can be usually evaluated, as described in detail in the Example, by using the ratio of bladder weight to body weight (bladder weight/body weight).

The nonbacterial prostatitis animal model of the present invention, having such characteristics histologically and pathologically similar to those of human chronic nonbacterial prostatitis and further preferably having the increased weight of the bladder, can be produced by injecting hydrochloric acid beneath the prostatic capsule of a nonhuman animal and rearing it for a specific period of time.

Examples of nonhuman animals for use in producing such an animal pathological model include mammals other than humans that are generally used in experiments, such as rats, nude rats, mice, nude mice, guinea pigs, hamsters, rabbits, dogs, cats, and monkeys. Preferable are rodents such as rats, nude rats, mice, nude mice, guinea pigs, hamsters, rabbits, etc. More preferable, for ease of operation and rearing, are rats, nude rats, mice, and nude mice. When such animals are, for example, rats, mice, nude mice and the like, it is preferable to use them when they are 10 to 12 weeks old.

The concentration of hydrochloric acid used in the present invention is, for example, 0.1 to 0.6 N, and preferably 0.2 to 0.5 N. Although the amount injected varies depending on the type of a subject nonhuman animal, it is usually 0.2 to 0.35 ml/kg body weight, and preferably 0.25 to 0.3 ml/kg body weight.

Methods for injecting hydrochloric acid beneath the prostatic capsule are not limited. As an example, administration by injection beneath the prostatic capsule using a disposable syringe equipped with a 27 G intravenous injection needle is convenient.

Hydrochloric acid is injected beneath the prostatic capsule of the aforementioned nonhuman animal. The specific injection site is preferably the lateral lobe of the prostate that is anatomically similar to the peripheral zone which is a predilection site of human prostatitis.

The nonhuman animal thus injected with hydrochloric acid beneath the prostate capsules is subjected to rearing. Rearing conditions are not limited. Ordinary rearing conditions are applicable as well as antibacterial conditions. As a specific example, 3 animals are housed per cage and supplied ad libitum with solid feed and with water from an automatic watering device under the conditions of 20 to 26°C, 30 to 70% relative humidity, 12-hour light-dark cycles, and at least 10 times/hour of ventilation frequency.

The rearing period for the nonhuman animal after hydrochloric acid injection is usually for about 3 days to about 2 weeks, and preferably for about 4 days to about 1 week. Thereby, the prostate tissue of the nonhuman animal that has received hydrochloric acid beneath the prostatic capsule develops pathohistological characteristics similar to those of the prostate tissue of a human suffering from chronic nonbacterial prostatitis. Moreover, the nonhuman animal exhibits lower urinary tract disorders (in particular, urine storage disorders such as pollakiuria, reduced volume of urine per urination, and increased residual urine) that are the pathology (symptoms) typically_ observable in human patients of chronic nonbacterial prostatitis.

The nonhuman animal thus prepared can be provided as a nonbacterial prostatitis animal model mimicking the pathology of human chronic nonbacterial prostatitis. An especially preferable nonbacterial prostatitis animal model mimicking the pathology of human chronic nonbacterial prostatitis is a nonhuman animal that has undergone 8 days of rearing after hydrochloric acid injection.

The nonbacterial prostatitis animal model can be effectively used in searching for substances effective for treating human chronic nonbacterial prostatitis and in evaluating the therapeutic effect of test substances and test drugs on human chronic nonbacterial prostatitis.

### II. Method for screening for an active ingredient for treating human chronic nonbacterial prostatitis

The present invention relates to a method for screening for a substance effective for treating human chronic nonbacterial prostatitis using the aforementioned nonbacterial prostatitis animal model that mimicks the pathology of human chronic nonbacterial prostatitis.

Specifically, this method can be practiced by administering a test substance to the aforementioned nonbacterial prostatitis animal model and examining the effect of the test substance for the amelioration of one or both of prostate tissue damage and lower urinary tract disorders of the nonbacterial prostatitis animal model.

Subject nonbacterial prostatitis animal models are not limited insofar as they are of the animals described above. Preferable examples are rats, nude rats, mice, and nude mice, with rats being particularly preferable. Those that have undergone at least 4 days, preferably about 4 days to about 1 week of rearing, and more preferably at least 8 days of rearing, after hydrochloric acid injection, are preferably used.

Test substances usable herein are not limited. Low molecular weight organic and inorganic compounds, high molecular weight organic and inorganic compounds, nucleic acids, amino acids, peptides, and proteins can be targeted. They may be purified products or crude products such as extracts and the like obtained from plants, animals, microorganisms, etc. Methods for preparing test substances are also not limited, and test substances may be isolated from natural products, may be chemically or biochemically synthesized, or may be genetically engineered.

Methods for administering test substances to the nonbacterial prostatitis animal model are not limited. Oral, subcutaneous, endodermic, intravenous, transdermal, enteral (rectal), and like parenteral administrations can be suitably selected according to the type of test substance to be administered, with oral administration being preferable.

The therapeutic effect of a test substance on human chronic nonbacterial prostatitis can be evaluated by examining the effect of the test substance for amelioration of the prostate tissue (or prostate and the tissue in the vicinity thereof) damage and lower urinary tract disorders of at least one disorder selected from the nonbacterial prostatitis animal model. Specifically, the evaluation of the therapeutic effect of a test substance can be practiced by administering a test substance to the animal model, rearing it for a certain period of time, observing the prostate tissue (or the prostate and the tissue in the vicinity thereof) of the animal model and evaluating the pathohistological characteristics thereof; or measuring the function of the bladder (in connection with voided volume, urination frequency, volume of residual urine, etc.) and evaluating the amelioration of tissue damage of the prostate tissue (or the prostate and the tissue in the vicinity thereof) or lower urinary tract disorders initially exhibited by the animal model. Although the screening method of the invention can be practiced by evaluating the amelioration of either the aforementioned tissue damage or lower urinary tract disorders, it is desirable to evaluate the amelioration of both the tissue damage and the lower urinary tract disorders.

In addition to the aforementioned examination, the bladder of the animal model can be weighed to use the effect of inhibiting bladder weight increase as an amelioration index.

The evaluation of a pathologic tissue can be carried out by preparing tissue sample slides by staining a target tissue with hematoxylin-eosin (H.E.) and observing the slides with an optical microscope at 20- to 100-times magnification. Specifically, the prostate tissue (or the prostate and the tissue in the vicinity thereof) of a test animal model is observed with an optical microscope, and its pathohistological change is examined in view of the organ (the prostate tissue (or the prostate and the tissue in the vicinity thereof)) of a corresponding normal nonhuman animal. In this instance, the pathological tissue of the test animal model can be evaluated according to the following criteria for pathological grading: 1: no change (no difference from the tissue of a normal nonhuman animal (normal tissue)), 2: insignificant (change recognizable only when compared with normal tissue), 3: slight (about 1/4 of the tissue is disordered); 4: moderate (about 1/2 of the tissue is disordered); and 5: severe (more than 1/2 of the tissue is clearly disordered). Effects for amelioration of tissue damage can be evaluated according to the change in the aforementioned (severity) grade (downward shift in the grade: ameliorative effect; upward shift in the grade: no ameliorative effect).

In the manner described above, test substances that ameliorating effect on prostate tissue damage and/or lower urinary tract disorders exhibited by the nonbacterial prostatitis animal model prior to the test substance administration and thereby bring the state of the prostate tissue (tissue image) and the bladder function thereof close to those of a corresponding normal nonhuman animal, and preferably the test substances that have an effect for inhibiting bladder weight increase, can be selected as effective substances for treating human chronic nonbacterial prostatitis.

Specifically, the selection of treatment effective substances as described above can be practiced by rearing in the same manner for a certain period of time control nonbacterial prostatitis model animals to which a test substance is not administered (a comparative animal), examining the tissue damage of the prostate tissue (or the prostate and the tissue in the vicinity thereof) and/or the function of the bladder, measuring the weight of the bladder as necessary, and comparing the resulting data with those of a test animal that received a test substance and reared for a certain period of time. As a result, test substances that give an ameliorative effect with respect to tissue damage, e.g., of the prostate, and/or the bladder function of the test animal, or that can bring the prostate tissue and/or the bladder function, and the weight of the bladder (ratio of bladder weight to body weight), of the test animal close to those of a normal nonhuman animal can be selected as treatment effective substances.

The substances thus selected can be advantageously used as active ingredients of therapeutic agents for human chronic nonbacterial prostatitis.

Among the substances effective in the treatment of human chronic nonbacterial prostatitis selected according to the aforementioned screening method of the invention, more effective and more practically usable treatment effective substances can be singled out as necessary by other efficacy tests, safety tests, etc., and further by clinical tests with human chronic nonbacterial prostatitis patients.

The treatment effective substances thus selected can be industrially produced by chemical synthesis, biochemical synthesis (fermentation), or genetic engineering based on their structural analysis.

### III. Method for screening for an active ingredient for preventing human chronic nonbacterial prostatitis

The present invention further relates to a method for screening for a substance effective for preventing human chronic nonbacterial prostatitis.

This method can be practiced by using a test animal that has not yet developed prostatitis, i.e., a preparatory nonbacterial prostatitis animal model, obtained during the process of preparing the aforementioned nonbacterial prostatitis animal model of the invention.

Specifically, the preparatory nonbacterial prostatitis animal model can be produced in the same manner as the aforementioned nonbacterial prostatitis animal model by injecting according to the method described above hydrochloric acid beneath the prostatic capsule, and preferably beneath the prostatic capsule of a dorsolateral lobe, of a nonhuman animal. The preparatory nonbacterial prostatitis animal model herein can be defined as a nonhuman animal that is in the condition, immediately or shortly after hydrochloric acid injection, of having not yet developed prostatitis.

The term "having not yet developed prostatitis" refers to a state in which tissue damage in the prostate tissue, or in the prostate and the tissue in the vicinity thereof, typically observed in the aforementioned nonbacterial prostatitis animal model, have not substantially developed, and in which lower urinary tract disorders have not substantially developed.

A nonhuman animal immediately after or within 4 days after hydrochloric acid injection can usually be used.

The method of the invention can be practiced by administering a test substance to the preparatory nonbacterial prostatitis model animal and examining the effect of the test substance for inhibiting the development of tissue damage of the prostate tissue (or the prostate and the tissue in the vicinity thereof) and/or for inhibiting the development of lower urinary tract disorders.

Examples of test substances and methods for administration thereof usable herein are as those described in (II) above.

The preventive effect of a test substance for human chronic nonbacterial prostatitis can be evaluated by examining the effect of inhibiting tissue damage of the prostate tissue (or the prostate and the tissue in the vicinity thereof) and/or effect of inhibiting lower urinary tract disorders of the preparatory nonbacterial prostatitis model animal. Specifically, the evaluation of the preventive effect of a test substance can be practiced by administering a test substance to the preparatory nonbacterial prostatitis model animal, rearing it for a specific period of time (about 1 week in the case of rats), examining the prostate tissue (or the prostate and the tissue in the vicinity thereof) and evaluating the pathohistological characteristics thereof; or measuring the bladder functions (in connection with the volume of urine per urination, urination frequency, volume of residual urine, etc.), and examining any development of tissue damage and lower urinary tract disorders that are observable in the nonbacterial prostatitis model animal. Although the screening method of the invention can be practiced by evaluating the presence or absence of the development of either aforementioned tissue damage or lower urinary tract disorders (development inhibitory effect), it is desirable to evaluate both of the tissue damage and the lower urinary tract disorders.

In addition to the aforementioned examination, the weight of the bladder is measured, and, if the weight of the bladder is not increased, this can be considered as an indicator of prevention.

The evaluation of a pathologic tissue can be carried out according to the method described in (II) above. The preventive effect for tissue damage can be evaluated according to the change in the aforementioned (severity) grading (no upward shift in the grade: preventive effect, upward shift in the grade: no preventive effect).

With respect to the test animal model (the preparatory nonbacterial prostatitis animal model), test substances that have inhibitory effect of inhibiting the development of tissue damage of the prostate tissue (or the prostate and the tissue in the vicinity thereof) and/or lower urinary tract disorders observable in the nonbacterial prostatitis animal model, and thereby have an effect of sustaining the condition of the prostate tissue (tissue image) and function of the bladder of the test animal model compared to those of a corresponding normal nonhuman animal, and preferably the test substance that have an effect of inhibiting an bladder weight increase, can be selected as substances effective for preventing human chronic nonbacterial prostatitis.

Specifically, the selection of prevention effective substances as described above can be practiced by rearing for a specific period of time in the same manner as above a control preparatory nonbacterial prostatitis model animals to which a test substance is not administered(a comparative animal), examining tissue damage of the prostate tissue (or the prostate and the tissue in the vicinity thereof) and/or the function of the bladder, and if necessary the weight of the bladder, and comparing the resulting data with those of a test animal that has received a test substance and reared for a specific period of time. As a result, test substances that have an effect of sustaining the prostate tissue and/or the function of the bladder of the test animal, and if necessary the weight of the bladder (ratio of bladder weight to body weight), of the test animal at the same or similar levels to those of normal nonhuman animals can be selected as prevention effective substances.

The substances thus selected can be advantageously used as active ingredients of preventive agents for human chronic nonbacterial prostatitis.

Among the substances effective for the prevention of human chronic nonbacterial prostatitis selected according to the aforementioned screening method of the invention, more effective and more practically usable prevention effective substances can be singled out as necessary by other efficacy tests, safety tests, etc., and further by clinical tests with human chronic nonbacterial prostatitis patients.

The prevention effective substances thus selected can be industrially produced by chemical synthesis, biochemical synthesis (fermentation), or genetic engineering based on their structural analysis.

### IV. Pharmaceutical compositions for prevention or treatment of human chronic nonbacterial prostatitis

Substances effective for preventing or treating human chronic nonbacterial prostatitis obtained according to the aforementioned screening methods can be used as such or as formulated into pharmaceutical compositions by mixing with known pharmaceutically acceptable carriers (including excipients, extenders, binders, lubricants, etc.) and conventional additives.

The present invention provides such pharmaceutical compositions for treating or preventing human chronic nonbacterial prostatitis. The active ingredients of the pharmaceutical compositions are not limited to those screened and directly obtained by the aforementioned methods, but include those chemically synthesized, biochemically synthesized, or genetically engineered based on the data obtained from the above-screened substances using conventional production methods.

The pharmaceutical compositions can be orally or parenterally administered according to the form thereof (tablet, pill, capsule, powder, granule, syrup, injection solution, drip fluid, externally-applied preparation, suppository, etc.). Although dosage cannot be specified because it depends on the type of active ingredient, administration route, administration subject, symptom, etc., it is about 0.0001 to about 5 g per day in single or divided doses.

### V. Method for evaluating the efficacy of a test drug for the amelioration of human chronic nonbacterial prostatitis

The present invention relates to a method for evaluating the efficacy of a test drug for ameliorating or treating human chronic nonbacterial prostatitis using the aforementioned nonbacterial prostatitis animal model mimicking the pathology of human chronic nonbacterial prostatitis.

Specifically, the method can be practiced by administering a test drug to the aforementioned nonbacterial prostatitis animal model, and evaluating the effect of the test drug for amelioration (treatment) of tissue damage of the prostate tissue (or the prostate and the tissue in the vicinity thereof) and/or lower urinary tract disorders of the nonbacterial prostatitis animal model.

Subject nonbacterial prostatitis animal models are the nonhuman animals described in (III) above.

It is sufficient that drugs usable herein as test drugs exhibit an effect for ameliorating (treating) human chronic nonbacterial prostatitis regardless of the strength thereof, and the drugs are therefore not limited to those having the form of pharmaceutical products. Hence, low molecular weight organic and inorganic compounds, high molecular weight organic and inorganic compounds, nucleic acids, amino acids, peptides, and proteins can be used. They may be purified products or crude products such as extracts and the like obtained from plants, animals, microorganisms, etc. Methods for obtaining such test drugs are not limited. They may be isolated from natural products, may be chemically or biochemically synthesized, or may be genetically engineered.

Methods for the administration of test drugs to the nonbacterial prostatitis animal model are not limited. Oral, subcutaneous, endodermic, intravenous, transdermal, enteral (rectal), and like parenteral administrations can be suitably selected according to the type of test drugs to be administered, with oral administration being preferable.

The efficacy of a test drug in the amelioration of human chronic nonbacterial prostatitis can be evaluated by examining the effect of the test drugs for ameliorating tissue damage of the prostate tissue (or the prostate and the tissue in the vicinity thereof) and/or lower urinary tract disorders of the nonbacterial prostatitis animal model. Specifically, the evaluation of the therapeutic effect of a test drug can be practiced by administering a test drug to the nonbacterial prostatitis animal model, rearing it for a certain period of time, examining the prostate tissue (or the prostate and the tissue in the vicinity thereof) of the model animal and evaluating the pathohistological characteristics thereof; or measuring the function of the bladder (in connection with urinary volume, urination frequency, volume of residual urine, etc.) and evaluating the extent of amelioration of tissue damage and lower urinary tract disorders of the animal model. Although it is desirable to evaluate the extent of amelioration of both tissue damage and lower urinary tract disorders, it is sufficient to evaluate the extent of amelioration of either tissue damage or lower urinary tract disorders.

In addition to the aforementioned examination, the bladder of the animal model can be weighed and the effect of inhibiting bladder weight increase may be used as an index in the efficacy evaluation.

The extent of amelioration of tissue damage can be evaluated according to the criteria for pathological grading given in (III) above: 1: no change (no difference from the tissue of a normal nonhuman animal (normal tissue)), 2: insignificant (change recognizable only when compared with normal tissue), 3: slight (about 1/4 of the tissue is disordered); 4: moderate (about 1/2 of the tissue is disordered); and 5: severe (more than 1/2 of the tissue is clearly disordered).

The evaluation of the efficacy of a test drug for amelioration of human chronic nonbacterial prostatitis can be practiced more conveniently when one or more drugs are used as comparative drugs whose effects for treating human chronic nonbacterial prostatitis and the strength thereof are known, and the ameliorative effects of the comparative drugs on the nonbacterial prostatitis animal model (ameliorative effects on tissue damage and lower urinary tract disorders and, if necessary, inhibitory effects on bladder weight increases) are compared with such effects of the test drug. Examples of drugs known to have therapeutic effects on human chronic nonbacterial prostatitis are new quinolones such as sparfloxacin (Spara) and levoflaxacin (Cravit); tetracycline-based antibacterial agents such as minocycline and doxycycline; nonsteroidal antiinflammatory agents such as ibuprofen and indomethacin; plant preparations such as cernilton, eviprostat, and paraprost), Chinese medicines such as cinnamon and hoelen formula, and *Shimotsu-to*.

### VI. Method for evaluating the efficacy of a test drug for the prevention of human chronic nonbacterial prostatitis

The present invention relates to a method for evaluating the efficacy of a drug for preventing human chronic nonbacterial prostatitis.

This method can be practice by using a test animal that has not yet developed prostatitis, i.e., a preparatory nonbacterial prostatitis animal model, obtained during the process of preparing the aforementioned nonbacterial prostatitis animal model of the invention described in (I) and (IV) above.

The method of the invention can be practiced by administering a test drug to the preparatory nonbacterial prostatitis model animal and evaluating the effect of the test drug for inhibiting (prevention) the development of tissue damage of the prostate tissue (or the prostate and the tissue in the vicinity thereof) and/or the development of lower urinary tract disorders.

It is sufficient that test drugs usable herein exhibit an effect for preventing (development inhibitory effect) human chronic nonbacterial prostatitis regardless of the strength thereof, and test drugs are therefore not limited to those having the form of pharmaceutical products. Hence, low molecular weight organic and inorganic compounds, high molecular weight organic and inorganic compounds, nucleic acids, amino acids, peptides, and proteins can be used. They may be purified products or crude products such as extracts and the like obtained from plants, animals, microorganisms, etc. Methods for obtaining such test drugs are not limited. They may be isolated from natural products, may be chemically or biochemically synthesized, or may be genetically engineered. Methods of their administration are as given in (V) above.

The efficacy of a test drug in the prevention of human chronic nonbacterial prostatitis can be evaluated as described above by examining the effect of the test drug for inhibiting the development of tissue damage of the prostate tissue (or the prostate and the tissue in the vicinity thereof) and/or lower urinary tract disorders of the preparatory nonbacterial prostatitis animal model. Specifically, the evaluation of the efficacy of a test drug for preventing human chronic nonbacterial prostatitis can be conducted by administering a test drug to the preparatory nonbacterial prostatitis animal model, rearing it for a certain period of time, examining the prostate tissue (or the prostate and the tissue in the vicinity thereof) of the animal model and evaluating the pathohistological characteristics thereof; or measuring the function of the bladder (in connection with the volume of urine per urination, urination frequency, volume of residual urine, etc.) and evaluating the extent of inhibiting the development of tissue damage and lower urinary tract disorders of the animal model. Although it is desirable to evaluate the extent of inhibiting the development of both tissue damage and lower urinary tract disorders, it is sufficient to evaluate the extent of inhibiting the development of either tissue damage or lower urinary tract disorders.

In addition to the aforementioned examination, the bladder of the animal model can be weighed and the effect of inhibiting bladder weight increase may be used as an index in the efficacy evaluation.

The extent of inhibition of the development of tissue damage can be evaluated according to the criteria for pathological grading given in (III) above: 1: no change (no difference from the tissue of a normal nonhuman animal (normal tissue)), 2: insignificant (change recognizable only when compared with normal tissue), 3: slight (about 1/4 of the tissue is disordered); 4: moderate (about 1/2 of the tissue is disordered); and 5: severe (more than 1/2 of the tissue is clearly disordered).

The evaluation of the efficacy of a test drug in the prevention of human chronic nonbacterial prostatitis can be practiced more conveniently when one or more drugs are used as comparative drugs whose effects for preventing human chronic nonbacterial prostatitis and the strength thereof are known, and the preventive effects of the comparative drugs on the preparatory nonbacterial prostatitis animal model (preventive effects on tissue damage and/or lower urinary tract disorders and, if necessary, inhibitory effects on bladder weight increases) are compared with such effects of the test drug. Examples of drugs known to have preventive effects on human chronic nonbacterial prostatitis are plant preparations such as cernilton, eviprostat, and paraprost; and Chinese medicines such as cinnamon and hoelen formula, and *Shimotsu-to.*

### EXAMPLES

Examples are given below to illustrate the invention in more detail. While these examples demonstrate embodiments of the invention, the scope of the invention is not limited by these examples.

### Example 1

### 1) Production of an animal model

### 1-1) Animal model for pathohistological evaluation

18 male Wistar rats at the age of 12-weeks-old were divided into 3 groups (test group, comparative group, and negative control group; 6 rats per group). The right inguinal region of each test group rat was incised under ether anesthesia to expose the prostate. Beneath the prostatic capsule of the right lateral lobe was injected 100 µl of sterilized 0.4 N hydrochloric acid physiological saline (a solution prepared by diluting hydrochloric acid with physiological saline to a concentration of 0.4 N, the same applies hereinbelow). The right inguinal region of each comparative group rat was incised in the same manner under ether anesthesia, and 100 µl of 0.4 N hydrochloric acid physiological saline was injected into the right vas deferens thus exposed. The peritoneum and the skin of the rats were sutured with a suture thread. To prevent operational infection, a kanamycin-containing aqueous solution was sprayed onto the site of the incision, and a physiological saline solution containing cefazolin sodium was injected into the femoral muscle in an amount of 7 mg/kg body weight. The control group refers to untreated rats (normal rats) subjected to neither an incisional operation nor hydrochloric acid injection.

### 1-2) Animal model for measuring the function of the bladder (presence/absence of lower urinary tract disorders)

9 male Wistar rats at the age of 12-weeks-old were divided into 3 groups (test groups: one group (n = 3) to be injected with 0.1 N hydrochloric acid, one group (n = 4) to be injected with 0.2 N hydrochloric acid, one group (n = 2) to be injected with 0.4 N hydrochloric acid). In the same manner as in 1-1) above, the right inguinal region of each rat was incised under ether anesthesia to expose the prostate. Beneath the prostatic capsule of the right lateral lobe was injected 100 µl of sterilized 0.1, 0.2 or 0.4 N hydrochloric acid physiological saline. For comparison, 100 µl of 0.4 N hydrochloric acid physiological saline was injected into the right vas deferens of 4 male Wistar rats at the age of 12 weeks old (comparative group). In addition, nonhydrochloric acid-injected rats (sham operation rats) were prepared (control group) by injecting, in place of the hydrochloric acid injected to the rats of the test groups, physiological saline beneath the capsule of the right lateral lobe of the prostate in the same amount as that of the hydrochloric acid. The peritoneum and the skin of these rats were sutured with a suture thread. To prevent operational infection, a kanamycin-containing aqueous solution was sprayed onto the site of the incision, and a physiological saline solution containing cefazolin sodium was injected into the femoral muscle in an amount of 7 mg/kg body weight.

The rats of the respective groups prepared in 1-1) and 1-2) were reared under the conditions described below:

### (Rearing conditions)

Three rats were housed per stainless steel rat bracket cage of R-IS type (size: 260 x 380 x 180 mm, manufactured by CLEA Japan Inc.)

Environment of the rearing facility: SPF (specific pathogen free) rearing facility at a temperature of 20 to 26°C, relative humidity of 30 to 70%, 12-hour light/dark cycles with light-on at 6 am and light-off at 6 pm, ventilation frequency of at least 10 times/hour; feeding and watering: ad libitum consumption of sterilized solid feed (CE-2, CLEA Japan Inc.) and tap water sterilized by an ultraviolet sterilizer and supplied by an automatic watering device.

### 2) Pathohistological evaluation

The prostate, bladder and urethra were sampled from the rats of respective groups prepared in 1-1) above (test group, comparative group, and control group) to prepare tissue specimens thereof for the evaluation of change in tissue.

Specifically, the rats of the test group and the comparative group were euthanized by exsanguination under ether anesthesia 6 hours (2 rats per group), 4 days (2 rats per group), or 8 days (2 rats per group) after hydrochloric acid injection. The prostate, bladder and urethral tissues were taken out as a lump, and the tissues were fixed with 4% paraformaldehyde. The rats of the control group were subjected to the same treatment.

The fixed tissues were treated with an OCT compound (Tissue-Tek O.C.T. compound (Sakura Finetechnical Co., Ltd.): 10.24 w/w% polyvinyl alcohol, 4.26 w/w% polyethylene glycol, 85.50 w/w% inactive components) to prepare frozen blocks. The frozen blocks were sliced in a cryostat to a thickness of 5 to 6 µm to prepare slide preparations of the tissue sections. The slide preparations were stained with hematoxylin-eosin (H.E), and the appearance of the tissues was observed under a microscope.

### 2-1) Rats injected with hydrochloric acid beneath the prostatic capsules

The observations of the prostate tissues of the rats which received 100 µl of 0.4 N hydrochloric acid physiological saline beneath the capsule of the right lateral lobe (test group: rats injected with hydrochloric acid beneath the prostatic capsules) obtained by observing/evaluating the prostate tissues 6 hours, 4 days, and 8 days after hydrochloric acid injection as well as the tissue observations of the corresponding control group rats (nonhydrochloric acid-injected rats) are presented in Figure 1: (observations when viewed along the longitudinal plane), and Figure 2: (observations when viewed along the transverse plane). In the figures, sample code N refers to the tissue samples obtained from the control group (nonhydrochloric acid-injected rats), and sample code P refers to the tissue samples obtained from the test group (rats injected with hydrochloric acid beneath the prostatic capsules).

As seen from the results, with respect to the prostate tissues of the rats which received 0.4 N hydrochloric acid into the prostate lateral lobes (sample code: P), slight (+) to moderate (++) to severe (+++) degrees of loss of glandular epithelial cells, peeling of epithelium, degeneration / necrosis, and presence of erythrocytes were observed at the glandular cavities of the prostate dorsolateral lobes 6 hours after hydrochloric acid injection (Figure 1(a), Figure 2(a)). Moreover, in the stroma of the prostate dorsolateral lobes, slight (+) to moderate (++) localized bleeding and nerve cell tigrolysis were observed (Figure 2(a)). Four days after hydrochloric acid injection, bleeding from the glandular cavities and their vicinity and the atrophy of the glandular cavities were observed, and moderate (++) to severe (+++) hyperplasia and fibrosis of the fibroblast, and inflammatory cellular infiltration (infiltration of macrophages, eosinophilic leucocytes, etc.) were observed in the stroma of the prostate dorsolateral lobes (Figure 1(b), Figure 2(b)). The rats of the test group prepared in 1-1) above developed severe prostate tissue damage as described above, and the damage were still observable 8 days after hydrochloric acid injection (Figure 1(c), Figure 2(c)). Figure 3 shows longitudinal sectional images (original: color pictures) of the prostatic lateral lobe and tissue in the vicinity of the urethra, and Figure 4 shows images (original: color pictures) of the lumen of the prostatic lateral lobe and the nerve cells, of a tissue sampled 8 days after hydrochloric acid injection. As seen from these results, the rats of test groups in which hydrochloric acid was injected beneath the prostatic capsules developed severe tissue damage exhibiting coagulation necrosis at the lumen wall affecting as far as a portion of prostate tissue near the urethra (Figure 3). Moreover, partial tissue disorder (tigrolysis) was observed in nerve cells attached to or present in the prostate (Figure 4).

With respect to the urethral and bladder tissues, there was no evidence of tissue damage extending from the prostate via the urethra. In view of this, it is considered that animal model of the invention (test group) has damage substantially only in the prostate tissue and presumably is purely a chemical substance-induced prostatitis model.

### 2-2) Rats injected with hydrochloric acid into the vas deferens (comparative group)

With respect to those rats injected with 100 µl of 0.4 N hydrochloric acid physiological saline into the vas deferens (comparative group), the observations of the prostate tissues, the glandular tissues of the ampulla ductus deferentis, and the tissues of the vas deferens, etc., obtained by observing/evaluating these tissues 6 hours, 4 days, and 8 days after hydrochloric acid injection, are presented in Figure 1 (observations when viewed along the longitudinal plane) and Figure 2 (observations when viewed along the transverse plane). In the figures, sample code Y refers to tissue samples obtained from the comparative group (rats injected with hydrochloric acid into the vas deferens). As seen from the results, with respect to those rats injected with 0.4 N hydrochloric acid physiological saline into the vas deferens, severe (+++) epithalaxia in the glandular cavities of the prostate dorsolateral lobes (see Figure 1(a)) and severe (+++) localized bleeding in the stroma (see Figure 2(a)) were observed at 6 hours after injection. However, the rats did not exhibit such prostate tissue damage 4 days after hydrochloric acid injection (see Figure 1(b) and Figure 2(b)).

Regarding the glandular tissues of the ampulla ductus deferentis, severe (+++) epithalaxia, degeneration/necrosis and bleeding in the glandular cavities (Figure 1(a)) and severe (+++) bleeding in the stromata (Figure 1(a), Figure 2(a)) were observed at 6 hours after hydrochloric acid injection. However, the rats at least 4 days after hydrochloric acid injection did not exhibit such damage at the glandular tissues of the ampulla ductus deferentis, and only a slight presence of erythrocytes suggesting bleeding and insignificant loss of epithelial cells in the glandular cavities, and slight cellular infiltration and insignificant fibrosis in the stromata were observed (Figure 1(b)(c), Figure 2(b)(c)).

### 3) Measurement of the function of the bladder (presence/absence of lower urinary tract disorder)

As described above in 1-2), 100 µl of sterilized 0.1, 0.2 or 0.4 N hydrochloric acid physiological saline was injected beneath the capsule of the right lateral lobe of the prostate of male Wistar rats at the age of 12 weeks old (3 groups), and the rats were reared for 8 days under the conditions described above. With respect to the rats thus treated (test groups), urination interval, volume of urine per urination, volume of residual urine, ratios of prostate ventral lobe weight, prostate dorsolateral lobe weight and bladder weight to body weight (prostate ventral lobe weight/body weight, prostate dorsolateral lobe weight/body weight, and bladder weight/body weight) were investigated to evaluate the function of the bladder (presence/absence of lower urinary tract disorders).

Specifically, each rat was weighed on the 8th day of rearing after hydrochloric acid injection and secured in the supine position under urethane anesthesia (1 g/kg i.p.) to shave the lower abdomen. After laparotomy, the bladder was exposed from the abdominal cavity. A small incision was given to the bladder dome,a polyethylene catheter (PE-50, Nippon Becton Dickinson Company, Ltd.) was inserted to the depth of about 6 mm, the incision was sutured, to fix the catheter. The tip of the catheter opposite to that inserted into and fixed in the bladder was divided by a three-way cock, and one opening thereof was connected to a pressure amplifier (NEC san-ei Instruments, 2238) via a transducer (a disposable life kit for blood pressure monitoring, DX-360, Nihon Kohden Corporation) to measure the inner pressure of the bladder, and the other opening thereof was connected via a physiological saline warmer (Blood Warmer TM-90, Toray Industries, Inc.) to a continuous infuser (Terufusion Syringe Pump STC-521, Terumo Corporation) to continuously introduce physiological saline into the bladder. The rats were secured in the prone position in a bolleman cage (Yamashitagiken, Co., Ltd.). Physiological saline maintained at about 37°C was continuous infused into the bladder at a rate of 5 ml/hr.

The waveform of the inner pressure of the bladder and voided volume were monitored for 30 minutes from 30 minutes after the beginning of the injection of physiological saline into the bladder until 60 minutes after the beginning of the injection of physiological saline into the bladder. The urination interval and volume of urine per urination were analyzed and evaluated based on the change in the waveform of the inner pressure and the weight or urine. The inner pressure of the bladder was recorded using a MacLab system (purpose-built analysis software, PowerLab/MacLac Chart v3.6s (AS Instruments Pty. Ltd.)). With respect to the voided volume, change in the weight of urine was recorded using a MacLab/4S system as above in a digital scale (HF-200, A&D Co., Ltd.) placed immediately below the cage by cummulatively trapping urine in a Petri dish.

Specifically, the urination interval can be measured as follows: When physiological saline is introduced into the bladder at a constant rate, the inner pressure thereof is gradually increased. When the amount of physiological saline exceeds a certain threshold, the inner pressure rapidly increases, whereby the inner pressure reaches a peak and induces urination. After urination, the inner pressure rapidly decreases (micturition reflex). Continual injection repeatedly induces such micturition reflex. The time interval between one peak of the inner pressure of the bladder ( maximum pressure) and another peak of the inner pressure of the bladder (maximum pressure) is referred to as the urination interval. The volume of urine per urination is computed from the reading of a digital scale provided immediately below the rats, which changes in relation to the weight of urine when urinating ( *Experimental methods for neurourological researches ,* Nihon-Igakukan Co., Ltd., 2000, 158-165). The volume of residual urine is calculated from the weight of the urine collected by suctioning the urine remaining in the bladder at the end of urination using a syringe.

After the aforementioned procedures, the prostate ventral lobes, prostate dorsolateral lobes, and bladders were excised, and the weight thereof measured.

As a control experiment, the function of the bladder (presence/absence of lower urinary tract disorders) was evaluated in the same manner as described above using the rats of the control group which were injected with 100 µl of physiological saline, in place of hydrochloric acid physiological saline, beneath the capsule of the right lateral lobe of the prostate of male Wistar rats at the age of 12 weeks old and reared for 8 days in the same manner as described above (sham operation rats).

The results are presented in Figure 5 (urination intervals, volumes of urine per urination), Figure 6 (volumes of residual urine, ratios of prostate ventral lobes weight to body weight), and Figure 7 (ratios of prostate dorsolateral lobe weight to body weight, ratios of bladder weight to body weight). The results show that the test groups (0.1 N hydrochloric acid-injected rats, 0.2 N hydrochloric acid-injected rats, 0.4 N hydrochloric acid-injected rats) exhibited significantly shorter urination intervals and smaller volumes of urine per urination compared with the control group (Figure 5). The test groups except for the 0.1 N hydrochloric acid-injected rats exhibited significantly higher volumes of residual urine compared with the control group (Figure 6). The test groups exhibited comparable or significantly higher ratios of prostate ventral lobe weight and bladder weight to body weight compared with the control group (Figures 6 and 7).

In view of the results of the measurement of bladder dysfunction conducted on the test groups, the following can be established:
First, since the bladder dysfunction lasted for a relatively long period of time even when a low concentration (0.1 N) of hydrochloric acid was used, the method of the present invention in which hydrochloric acid is injected into the prostate lateral lobe greatly affects the bladder function. Second, since parametric values for the bladder function in the test groups differ depending on the concentration of hydrochloric acid injected, purpose-built nonbacterial prostatitis animal models can be prepared by adjusting the concentration of hydrochloric acid. For example, 0.1 N hydrochloric acid-injected rats can be a nonbacterial prostatitis model predominantly for irritative bladder symptoms because 0.1 N hydrochloric acid-injected rats are considered to have no residual urine in view of the measurement results that are comparable to those of the control group rats, and 0.1 N hydrochloric acid-injected rats exhibited the shortest urination interval and the smallest volume of urine per urination. 0.2 N hydrochloric acid-injected rats can be considered as a nonbacterial prostatitis model accompanied by lower urinary tract disorders because they showed some residual urine and, although not identical to those of the 0.1 N hydrochloric acid-injected rats, their urination intervals are shorter and their volumes of urine per urination are smaller, compared with those of the rats of the control group. 0.4 N hydrochloric acid-injected rats can be considered as a nonbacterial prostatitis model of advanced lower urinary tract disorders because they exhibited the greatest volumes of residual urine, and the volumes of urine per urination thereof were smaller than those of the 0.2 N hydrochloric acid-injected rats.

In view of the ratios of prostate (ventral lobe and lateral lobe) weight and bladder weight to body weight, the weight of the bladders in the test groups (the animal model of the invention) increased in accordance with the concentration of hydrochloric acid injected. However, no evidence of correlation was found between the concentration of hydrochloric acid and the change in the weight of the prostate (weight increase of the prostate (prostatic hyperplasia) or prostate atrophy). It can therefore be presumed that, in relation to the concentration of hydrochloric acid injected, the extent of organic change in the prostate tissue as well as the effect on the nervous system intrinsic to the prostate change, and the change in the extent of lower urinary tract disorders (change in the volume of residual urine) is reflected in the weight of the bladder.

### INDUSTRIAL APPLICABILITY

The nonbacterial prostatitis animal model (a nonhuman animal) provided by the present invention is a pathological animal model mimicking the pathology of human chronic nonbacterial prostatitis in terms of pathohistological change in the prostate and presence/absence of lower urinary tract disorders, and preferably in terms of increase in the weight of the bladder. The animal model can be effectively used in searching and screening for substances effective for preventing or treating human chronic nonbacterial prostatitis. Furthermore, the animal model can be effectively used in evaluating the efficacy of a preventive or therapeutic agent for human chronic nonbacterial prostatitis. In other words, the nonbacterial prostatitis animal model (a nonhuman animal) provided by the present invention can be effectively used in developing a preventive or therapeutic agent for human chronic nonbacterial prostatitis.

## Claims

1. A nonbacterial prostatitis animal model exhibiting a prostate tissue damage characteristically observed in human chronic nonbacterial prostatitis and a lower urinary tract disorder characteristically observed in human chronic nonbacterial prostatitis,
the animal model being a nonhuman animal, and being prepared by injecting hydrochloric acid beneath the prostatic capsule.

2. The nonbacterial prostatitis animal model according to claim 1 having an increased ratio of bladder weight to body weight compared with a normal nonhuman animal.

3. The nonbacterial prostatitis animal model according to claim 1, wherein the lower urinary tract disorder is a urine storage disorder posing at least one symptom selected from the group consisting of pollakiuria, urinary incontinence, and reduced effective bladder capacity.

4. The nonbacterial prostatitis animal model according to claim 1, wherein the animal model is a nonhuman animal, and prepared by injecting hydrochloric acid beneath the capsule of a dorsolateral lobe.

5. The nonbacterial prostatitis animal model according to claim 1, wherein the animal model is a nonhuman animal reared for about 4 days to about 1 week after injection of hydrochloric acid beneath the prostatic capsule.

6. The nonbacterial prostatitis animal model according to claim 1, wherein the nonhuman animal is a rat.

7. A method for preparing the nonbacterial prostatitis animal model recited in claim 1 comprising the steps of:
injecting hydrochloric acid beneath the prostatic capsule of a nonhuman animal, and
rearing the nonhuman animal to develop prostatitis.

8. The method for preparing the nonbacterial prostatitis animal model according to claim 7, wherein hydrochloric acid is injected beneath the prostatic capsule of a dorsolateral lobe.

9. The method for preparing the nonbacterial prostatitis animal model according to claim 7 comprising the steps of:
injecting hydrochloric acid beneath the prostatic capsule, and
rearing the nonhuman animal for 4 days to 1 week.

10. The method for preparing the nonbacterial prostatitis animal model according to claim 7, wherein the nonhuman animal is a rat.

11. A method for screening for a substance for treating human chronic nonbacterial prostatitis comprising the steps of:
administering a test substance to the nonbacterial prostatitis animal model recited in any one of claims 1 to 6, and
examining the effect of the test substance for ameliorating at least one disorder selected from the group consisting of a prostate tissue damage and a lower urinary tract disorder of the nonbacterial prostatitis animal model.

12. The method for screening for a substance for treating human chronic nonbacterial prostatitis according to claim 11, wherein the lower urinary tract disorder is a urine storage disorder posing at least one symptom selected from the group consisting of pollakiuria, urinary incontinence, and reduced effective bladder capacity.

13. A method for screening for a substance for preventing human chronic nonbacterial prostatitis comprising the steps of:
administering a test substance to a preparatory nonbacterial prostatitis animal model prepared by injecting hydrochloric acid beneath the prostatic capsule, which has not yet developed prostatitis, and
examining the effect of the test substance for inhibiting the development of at least one disorder selected from the group consisting of a prostate tissue damage and a lower urinary tract disorder of the preparatory nonbacterial prostatitis animal model.

14. The method for screening for a substance for preventing human chronic nonbacterial prostatitis according to claim 13, wherein the preparatory nonbacterial prostatitis animal model is a nonhuman animal, and prepared by injecting hydrochloric acid beneath the prostatic capsule of the nonhuman animal and rearing the nonhuman animal for less than 4 days, which has not yet developed prostatitis.

15. The method for screening for a substance for preventing human chronic nonbacterial prostatitis according to claim 14, wherein the nonhuman animal is a rat.

16. A method for evaluating the efficacy of a drug for ameliorating human nonbacterial prostatitis, the method comprising the steps of:
administering a test drug whose efficacy is to be evaluated to the nonbacterial prostatitis animal model recited in any one of claims 1 to 6, and
examining the effect of the test drug for ameliorating at least one disorder selected from the group consisting of a prostate tissue damage and a lower urinary tract disorder of the nonbacterial prostatitis animal model.

17. The method for evaluating the efficacy of a drug for ameliorating human nonbacterial prostatitis according to claim 16, wherein the lower urinary tract disorder is a urine storage disorder posing at least one symptom selected from the group consisting of pollakiuria, urinary incontinence, and reduced effective bladder capacity.

18. A method for evaluating the efficacy of a drug for preventing human nonbacterial prostatitis, the method comprising the steps of:
administering a test drug whose efficacy is to be evaluated to a preparatory nonbacterial prostatitis animal model produced by injecting hydrochloric acid beneath the prostatic capsule, which has not yet developed prostatitis, and
examining the effect of the test drug for inhibiting the development of at least one disorder selected from group consisting of a prostate tissue damage and a lower urinary tract disorder of the preparatory nonbacterial prostatitis animal model.

19. A method for evaluating the efficacy of a drug for preventing human nonbacterial prostatitis according to Claim 18, wherein the preparatory nonbacterial prostatitis animal model is a nonhuman animal, and prepared by injecting hydrochloric acid beneath the prostatic capsule of a nonhuman animal and rearing the nonhuman animal for less than 4 days, which has not yet developed prostatitis.

20. A method for evaluating the efficacy of a drug for preventing human nonbacterial prostatitis according to Claim 19, wherein the lower urinary tract disorder is a urine storage disorder posing at least one symptom selected from the group consisting of pollakiuria, urinary incontinence, and reduced effective bladder capacity.

21. Use of the nonbacterial prostatitis animal model recited in any one of claims 1 to 6 to search for an active ingredient for a pharmaceutical composition for treating human nonbacterial prostatitis or to evaluate the efficacy of a pharmaceutical agent for treating human nonbacterial prostatitis.

22. Use of a preparatory nonbacterial prostatitis animal model prepared by injecting hydrochloric acid beneath the prostatic capsule, which has not yet developed prostatitis, to search for an active ingredient for a pharmaceutical composition for preventing human nonbacterial prostatitis or to evaluate the efficacy of a pharmaceutical agent for preventing human nonbacterial prostatitis.

23. The use according to claim 22, wherein the preparatory nonbacterial prostatitis animal model is a nonhuman animal, and prepared by injecting hydrochloric acid beneath the prostatic capsule of a nonhuman animal and rearing the nonhuman animal for less than 4 days.

24. A pharmaceutical composition for treating or preventing human nonbacterial prostatitis comprising a substance evaluated as having an effect for ameliorating or preventing nonbacterial prostatitis by the screening method recited in claims 11 or 14.
